# EUROPEAN PATENT APPLICATION

(11) **EP 3 042 691 A1**
(43) Date of publication of application: **13.07.2016**
(21) Application number: 14842202.5
(22) Date of filing: 05.09.2014
(51) Int. Cl.: A61M 39/10, A61J 15/00

(54) **DOUBLE MALE CONNECTOR**

(30) Priority: 06.09.2013 JP 2013184739
(71) Applicant: JMS Co., Ltd., Hiroshima-shi, Hiroshima 730-8652 (JP)
(72) Inventor: KUNISHIGE, Takahiko, Hirohima-shi, Hiroshima 730-8652 (JP); UEHARA, Yasumasa, Hirohima-shi, Hiroshima 730-8652 (JP); UEDA, Asami, Hirohima-shi, Hiroshima 730-8652 (JP); UEDA, Takayuki, Hirohima-shi, Hiroshima 730-8652 (JP); UEDA, Yutaka, Hirohima-shi, Hiroshima 730-8652 (JP); TAKIMOTO, Kazuhiko, Hirohima-shi, Hiroshima 730-8652 (JP); TOYOTA, Koichiro, Hirohima-shi, Hiroshima 730-8652 (JP); UEHARA, Megumi, Hirohima-shi, Hiroshima 730-8652 (JP)
(74) Representative: Piésold, Alexander James
(86) International application number: PCT/JP2014/073484
(87) International publication number: WO 2015/034045

(57) **Abstract**

A double male connector (1) includes a first male luer (10) on one end and a second male luer (20) on the other end. The first male luer (10) and the second male luer (20) are in communication with each other. An outer circumferential surface of the first male luer (10) constitutes a first tapered surface (12) whose outer diameter decreases toward a leading end of the first male luer, and an outer circumferential surface of the second male luer (20) constitutes a second tapered surface (22) whose outer diameter decreases toward a leading end of the second male luer. A tubular portion (15) surrounds the first male luer (10). A female screw (16) is formed on an inner circumferential surface of the tubular portion (15).

## Description

### Technical Field

The present invention relates to a double male connector having male luers on both ends, and particularly relates to a double male connector that is used to connect female connectors on respective ends of two tubes for use in enteral nutrition or the like.

### Background Art

Enteral nutrition is known as a method for parenteral administration of nutrients or drugs to patients. In enteral nutrition, a liquid substance (generally called "enteral formula") such as a nutritional formula, a liquid diet, or a drug is administered to a patient through a transnasal catheter inserted into the stomach or even the duodenum via the nasal cavity of the patient or a PEG (Percutaneous Endoscopic Gastrostomy) catheter inserted into a gastrostomy opening formed in the abdomen of the patient. The liquid substance to be administered to the patient is stored in a container. A feeding set constituted by a pliable tube is connected to an outlet port of the container. A downstream end of the feeding set is connected to an upstream end of the transnasal catheter or the PEG catheter (hereinafter these catheters are generically referred to as "the catheter") that is to be inserted into the patient. In general, a male connector is provided on the downstream end of the feeding set. Meanwhile, a female connector that is connectable to this male connector is provided on the upstream end of the catheter. In practice, connectors having various shapes are used as such male connectors and female connectors (see Patent Document 1, for example).

Incidentally, in the case where a liquid substance that is administered in enteral nutrition is a liquid having low viscosity, a problem may arise in which the liquid substance refluxes from the stomach to the esophagus resulting in the development of pneumonia as a complication, or water in the liquid substance is not sufficiently absorbed by the body thus causing diarrhea, for example. To address this issue, in enteral nutrition, the viscosity of the liquid substance is often increased (i.e., the liquid substance is semi-solidified) by adding a thickening agent or a bodying agent thereto, for example. The liquid substance whose viscosity is thus increased has low fluidity and therefore exhibits high resistance when passing through a tube. For this reason, during administration of the liquid substance to the patient, the liquid substance is pressure-fed by applying a pressure thereto.

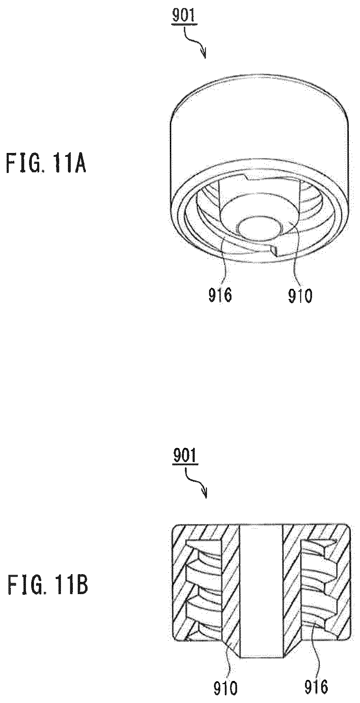

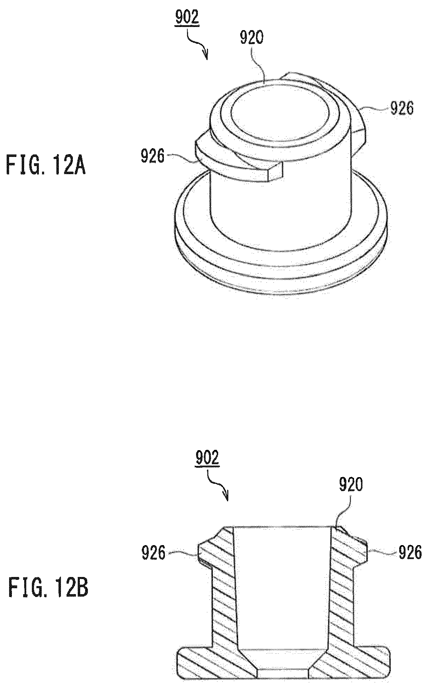

Thus, it is desirable that the connectors for connecting the feeding set and the catheter to each other include mutually engageable lock mechanisms so as to be able to withstand the pressure applied to the liquid substance. Under these circumstances, international standardization of male connectors and female connectors for use in such applications as an international standard ISO 80369-3 referring to medical devices for feeding systems has been considered. As shown in FIGS. 11A and 11B, a male connector 901 that is under consideration as ISO 80369-3 has a female screw 916 surrounding a tubular male luer 910. On the other hand, as shown in FIGS. 12A and 12B, a female connector 902 that is under consideration as ISO 80369-3 has a male screw 926 on an outer circumferential surface of a cylindrical insertion portion (female luer) 920 into which the male luer 910 is to be inserted. The male connector 901 and the female connector 902 are connected to each other by inserting the male luer 910 into the insertion portion 920 and screwing the male screw 926 into the female screw 916. The male connector 901 and the female connector 902 together provide a connection that has excellent liquid-tightness (the property of preventing the liquid substance from leaking through a connecting portion between the male connector and the female connector, even when pressure is applied to the liquid substance) and connection strength (the property of preventing disconnection of the male connector and the female connector that have been connected to each other, even when a pulling force is applied thereto).

If the above-described male connector 901 and female connector 902 are adopted as an international standard, it is highly likely that a feeding set equipped with the female connector 902 on its downstream end and a catheter equipped with the male connector 901 on its upstream end will be put to practical use.

### Citation List

### Patent Document

Patent Document 1: WO 2008/152871

### Disclosure of Invention

### Problem to be Solved by the Invention

However, it is expected that even after ISO 80369-3 becomes an international standard, there will be cases where catheters having conventional female connectors that are not compliant with ISO 80369-3 are still being used in real medical practices. Accordingly, there is a demand that a feeding set having the ISO 80369-3 compliant female connector 902 on its downstream end can be connected to a catheter having a conventional female connector on its upstream end.

It is an object of the present invention to provide a double male connector that enables an ISO 80369-3 compliant female connector to be connected to a conventional female connector.

### Means for Solving Problem

A double male connector of the present invention includes a first male luer on one end and a second male luer on the other end. The first male luer and the second male luer are in communication with each other. An outer circumferential surface of the first male luer constitutes a first tapered surface (conical surface) whose outer diameter decreases toward a leading end of the first male luer. An outer circumferential surface of the second male luer constitutes a second tapered surface (conical surface) whose outer diameter decreases toward a leading end of the second male luer. The double male connector further includes a tubular portion surrounding the first male luer. A female screw is formed on an inner circumferential surface of the tubular portion.

### Effects of the Invention

According to the present invention, the first male luer and the female screw surrounding the first male luer can be connected to an ISO 80369-3 compliant female connector, and also the second male luer can be connected to a conventional female connector. Therefore, for example, a feeding set having an ISO 80369-3 compliant female connector on its downstream end and a catheter having a conventional female connector on its upstream end can be connected to each other via the double male connector of the present invention.

### Brief Description of Drawings

[FIG. 1] FIG. 1A is a perspective view of a double male connector according to Embodiment 1 of the present invention when viewed from a first male luer side. FIG. 1B is a side view of the double male connector when viewed from a second male luer side. FIG. 1C is a cross-sectional view of the double male connector taken along a plane containing a central axis thereof.
[FIG. 2] FIG. 2A is a perspective view of a first female connector that is to be connected to the first male luer of the double male connector according to Embodiment 1 of the present invention. FIG. 2B is a cross-sectional view of the first female connector taken along a plane containing a central axis thereof.
[FIG. 3] FIG. 3A is a perspective view of a second female connector that is to be connected to the second male luer of the double male connector according to Embodiment 1 of the present invention. FIG. 3B is a cross-sectional view of the second female connector taken along a plane containing a central axis thereof.
[FIG. 4] FIGS. 4A to 4C are perspective views sequentially illustrating steps of a process for connecting the first female connector and the second female connector to each other via the double male connector according to Embodiment 1 of the present invention.
[FIG. 5] FIG. 5A is a perspective view of a double male connector according to Embodiment 2 of the present invention when viewed from a first male luer side. FIG. 5B is a side view of the double male connector when viewed from a second male luer side. FIG. 5C is a cross-sectional view of the double male connector taken along a plane containing a central axis thereof.
[FIG. 6] FIG. 6A is a perspective view of a second female connector that is to be connected to the second male luer of the double male connector according to Embodiment 2 of the present invention. FIG. 6B is a cross-sectional view of the second female connector taken along a plane containing a central axis thereof.
[FIG. 7] FIG. 7A is a perspective view of a handle constituting the second female connector of Embodiment 2 of the present invention. FIG. 7B is a top view of the handle. FIG. 7C is a front view of the handle.
[FIG. 8] FIGS. 8A and 8B are perspective views sequentially illustrating steps of a process for connecting the first female connector and the second female connector to each other via the double male connector according to Embodiment 2 of the present invention.
[FIG. 9] FIGS. 9A and 9B are perspective views sequentially illustrating steps of the process for connecting the first female connector and the second female connector to each other via the double male connector according to Embodiment 2 of the present invention.
[FIG. 10] FIG. 10A is a perspective view of another double male connector according to Embodiment 2 of the present invention when viewed from the first male luer side. FIG. 10B is a front view of the other double male connector.
[FIG. 11] FIG. 11A is a perspective view of a male connector that is under consideration as ISO 80369-3. FIG. 11B is a cross-sectional view of the male connector taken along a plane containing a central axis thereof.
[FIG. 12] FIG. 12A is a perspective view of a female connector that is under consideration as ISO 80369-3. FIG. 12B is a cross-sectional view of the female connector taken along a plane containing a central axis thereof.

### Description of the Invention

It is preferable that the first tapered surface and the female screw of the above-described double male connector of the present invention are compliant with ISO 80369-3. With this configuration, the double male connector of the present invention can be connected to an ISO 80369-3 compliant female connector in compliance with ISO 80369-3.

It is preferable that the entirety of the double male connector of the present invention is integrally formed as one component. With this configuration, the double male connector is easy to produce. Moreover, the strength and durability of the double male connector are improved.

It is preferable that the double male connector further includes a pair of engaging claws protruding outward between the first male luer and the second male luer. With this configuration, it is possible to engage the pair of engaging claws with an engagement structure provided on a female connector.

It is preferable that when the second male luer is inserted into a female connector, the pair of engaging claws engage with the female connector. With this configuration, the strength of the connection between the second male luer and the female connector can be improved.

Hereinafter, the present invention will be described in detail while showing preferred embodiments thereof. However, it goes without saying that the present invention is not limited to the embodiments below. In the drawings that will be referred to in the following description, only the main members of constituent members of the embodiments of the present invention that are necessary for the description of the present invention are shown in a simplified manner for the sake of convenience of description. Accordingly, the present invention may include optional members that are not shown in the drawings below. Moreover, it should be understood that the dimensions of the members in the drawings below are not faithful representations of the dimensions of actual members, dimensional ratios of those members, and the like.

### Embodiment 1

### Configuration of Double Male Connector

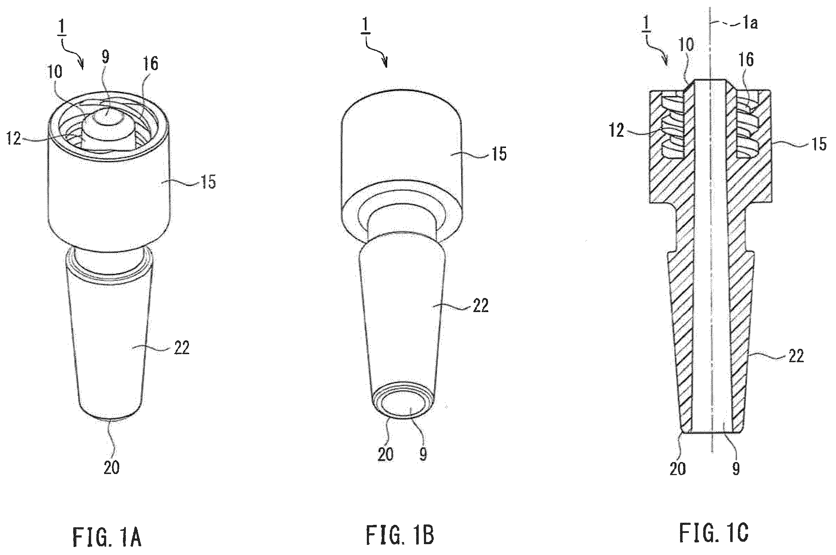

FIGS. 1A and 1B are perspective views of a double male connector 1 according to Embodiment 1 of the present invention. FIG. 1C is a cross-sectional view of the double male connector 1 taken along a plane containing a central axis 1a thereof.

The double male connector 1 includes a tubular, first male luer 10 on one end and a tubular, second male luer 20 on the other end. The double male connector 1 is a male-male connector with male connectors on both ends.

An outer circumferential surface of the first male luer 10 constitutes a tapered surface (first tapered surface) 12 whose outer diameter decreases toward a leading end of the first male luer 10. A tubular portion 15 is provided surrounding the first male luer 1. A female screw 16 is formed on an inner circumferential surface (surface that faces the first male luer 10) of the tubular portion 15. Although there is no limitation on the shape (outer diameter, taper angle, and the like) of the first tapered surface 12 and the shape (diameter, pitch, and the like) of the female screw 16, it is preferable that these are compliant with a male connector (see FIGS. 11A and 11B) of ISO 80369-3.

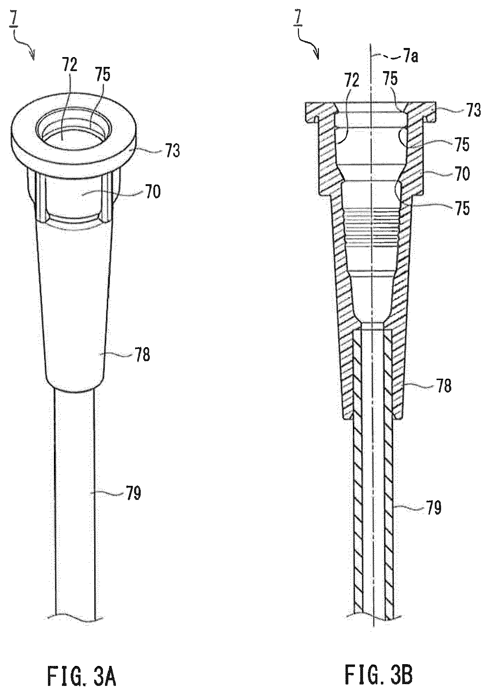

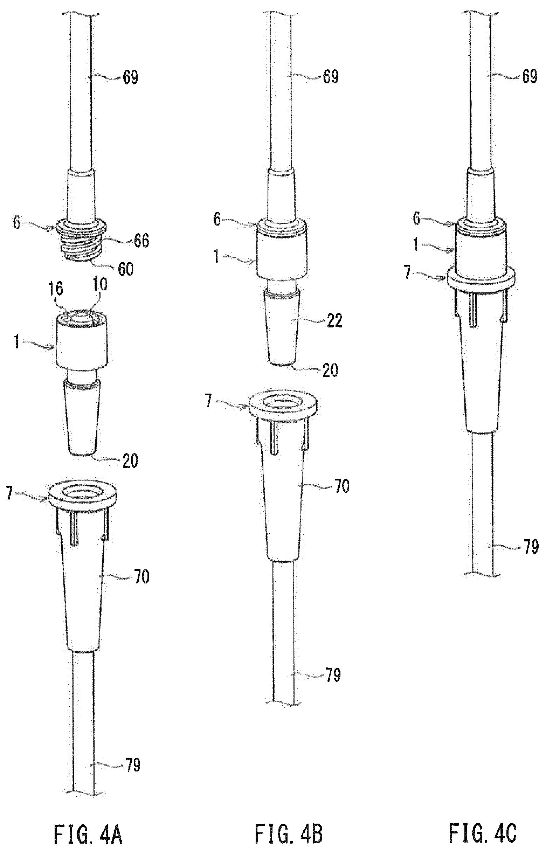

An outer circumferential surface of the second male luer 20 also constitutes a tapered surface (second tapered surface) 22 whose outer diameter decreases toward a leading end of the second male luer 20. Although there is no limitation on the shape (outer diameter, taper angle, and the like) of the second tapered surface 22, it is preferable that it has compatibility with a conventional male luer (see FIG. 4 of Patent Document 1, for example) that is adapted to a female luer (see FIGS. 3A and 3B) below, for example.

A flow channel 9 passes through the double male connector 1 along the central axis 1a. As a result, the first male luer 10 and the second male luer 20 are in communication with each other via the flow channel 9.

Although there is no limitation on the material for the double male connector 1, it is preferable that the double male connector 1 is made of a hard material having a degree of stiffness that is high enough for that material to be regarded as being substantially undeformable. For example, a resin material such as polyacetal, polycarbonate, polystyrene, polyamide, polypropylene, polyethylene, unplasticized polyvinyl chloride, or ABS (acryl-butadiene-styrene copolymer) can be used. Although the double male connector 1 can be formed by combining separately produced members together, it is preferable that the entirety of the double male connector 1 is integrally formed as one component by using injection molding or other methods because this improves the ease of manufacturing, strength, and durability of the double male connector 1. It should be noted that "integrally formed" as used in the present invention includes the cases where the double male connector 1 is produced by using coinjection molding, but does not include the cases where a plurality of components that have been separately produced are integrated by fitting, fusion bonding, or using an adhesive or the like.

### First Female Connector to be Connected to Double Male Connector

An example of a first female connector that is to be connected to the double male connector 1 will be described.

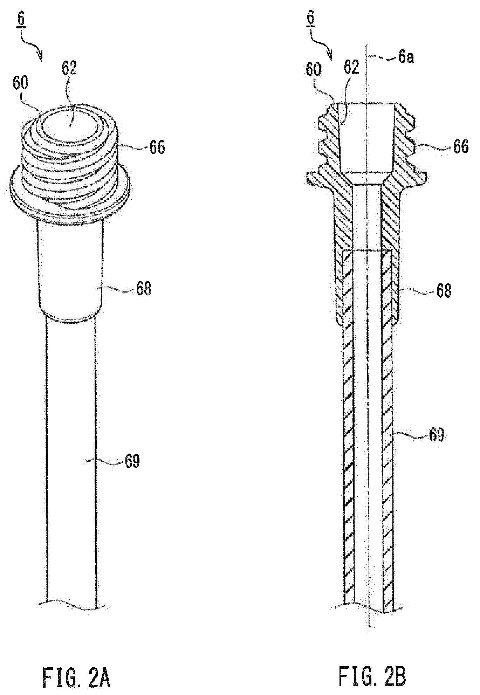

FIG. 2A is a perspective view of a first female connector 6 that is to be connected to the first male luer 10 of the double male connector 1. FIG. 2B is a cross-sectional view of the first female connector 6 taken along a plane containing a central axis 6a thereof.

The first female connector 6 includes on one end a cylindrical insertion portion 60 into which the first male luer 10 is to be inserted. An inner circumferential surface of the insertion portion 60 constitutes a tapered surface 62 whose inner diameter increases toward a leading end of the insertion portion 60. A male screw 66 is formed on an outer circumferential surface of the insertion portion 60. The shape (inner diameter, taper angle, and the like) of the tapered surface 62 and the shape (diameter, pitch, and the like) of the male screw 66 are compliant with a female connector (see FIGS. 12A and 12B) of ISO 80369-3.

The other end of the first female connector 6 constitutes a base end portion 68 to which a pliable tube 69 is connected. Although there is no limitation on the method for connecting the base end portion 68 and the tube 69 to each other, any method can be used, such as a method that uses an adhesive or a method that uses thermal fusion bonding.

Although there is no limitation on the material for the first female connector 6, it is preferable that the first female connector 6 is made of a hard material having a degree of stiffness that is high enough for that material to be regarded as being substantially undeformable. For example, a resin material such as polyacetal, polycarbonate, polystyrene, polyamide, polypropylene, polyethylene, unplasticized polyvinyl chloride, or ABS (acryl-butadiene-styrene copolymer) can be used.

The first female connector 6 and the tube 69 constitute a portion of a feeding set for use in enteral nutrition. The first female connector 6 serves as a female connector provided on a downstream end of the feeding set. A container, a syringe, or the like in which a liquid substance such as a nutritional formula, for example, is stored is connected to an end (upstream end) of the tube 69 opposite to the first female connector 6.

### Second Female Connector to be Connected to Double Male Connector

An example of a second female connector that is to be connected to the double male connector 1 will be described.

FIG. 3A is a perspective view of a second female connector 7 that is to be connected to the second male luer 20 of the double male connector 1, and FIG. 3B is a cross-sectional view of the second female connector 7 taken along a plane containing a central axis 7a thereof.

The second female connector 7 includes on one end a cylindrical insertion portion 70 into which the second male luer 20 is to be inserted. An inner circumferential surface of the insertion portion 70 constitutes a tapered surface 72 whose inner diameter increases toward a leading end of the insertion portion 70.

A plurality of ribs 75 protrude from the tapered surface 72 toward the central axis 7a, the ribs 75 each being continuous in a circumferential direction. The ribs 75 locally come into close contact with the second tapered surface 22 of the second male luer 20, thereby improving the liquid-tightness and the connection strength. The number of ribs 75 may be set at any number. Moreover, the ribs 75 may be omitted.

A flange 73 that is continuous in the circumferential direction protrudes from an opening end of the insertion portion 70 in an outward radial direction. The flange 73 helps a worker to grip the insertion portion 70 when inserting the second male luer 20 into the insertion portion 70. Moreover, the flange 73 improves the strength of the opening end of the insertion portion 70 and thus prevents the opening end of the insertion portion 70 from being deformed by the inserted second male luer 20.

The other end of the second female connector 7 constitutes a base end portion 78 to which a pliable tube 79 is connected. Although there is no limitation on the method for connecting the base end portion 78 and the tube 79 to each other, any method can be used, such as a method that uses an adhesive or a method that uses thermal fusion bonding.

It is preferable that the second female connector 7 is made of a material that is more pliable than the second male luer 20. Although there is no limitation on the material for the second female connector 7, it is preferable that the second female connector 7 is made of a soft material (generally also called "elastomer") having pliability (flexibility) and rubber elasticity. For example, rubber, such as natural rubber, isoprene rubber, or silicone rubber, or a thermoplastic elastomer, such as a styrene-based elastomer, an olefin-based elastomer, or a polyurethane-based elastomer, can be used. The second female connector 7 and the tube 79 may also be integrally formed using the same material.

The second female connector 7 and the tube 79 constitute a portion of a catheter, such as a PEG catheter or a transnasal catheter that have conventionally been used in enteral nutrition. The second female connector 7 serves as a female connector (see Patent Document 1) provided on an upstream end of the catheter. An end (downstream end) of the tube 79 opposite to the second female connector 7 is to be inserted into a patient.

### Manner in which Double Male Connector is Used

As shown in FIG. 4A, the double male connector 1 is used to connect the first female connector 6 provided on the downstream end of the feeding set and the second female connector 7 provided on the upstream end of the catheter to each other via the double male connector 1. Hereinafter, a manner in which the double male connector 1 is used will be described.

First, the double male connector 1 and the first female connector 6 are connected to each other. That is to say, the first male luer 10 of the double male connector 1 is inserted into the insertion portion 60 of the first female connector 6. Then, the double male connector 1 is rotated relative to the first female connector 6 to screw the male screw 66 of the first female connector 6 into the female screw 16 of the double male connector 1. The shape of the first tapered surface 12 (see FIG. 1C) of the first male luer 10 conforms to the shape of the tapered surface 62 (see FIG. 2B) of the first female connector 6, and thus the first tapered surface 12 and the tapered surface 62 come into close contact with each other. Therefore, the double male connector 1 and the first female connector 6 are connected to each other in a liquid-tight manner as shown in FIG. 4B by tightly screwing the male screw 66 into the female screw 16.

Next, the second male luer 20 of the double male connector 1 is inserted into the insertion portion 70 of the second female connector 7. Since the insertion portion 70 has rubber elasticity, the insertion portion 70 is widened and stretched in the circumferential direction by the second tapered surface 22 of the second male luer 20 as the second male luer 20 is inserted into the insertion portion 70. As shown in FIG. 4C, when the second male luer 20 is inserted sufficiently deep into the insertion portion 70, the elastic force of the insertion portion 70 that is stretched in the circumferential direction allows the insertion portion 70 to come into close contact with the second tapered surface 22 of the second male luer 20. Thus, the double male connector 1 and the second female connector 7 are connected to each other in a liquid-tight manner.

Conversely, it is also possible to first connect the double male connector 1 and the second female connector 7 to each other and then connect the double male connector 1 and the first female connector 6 to each other. However, with this connection method, in a state in which the first female connector 6 and the second female connector 7 are connected to each other via the double male connector 1 as shown in FIG. 4C, the tubes 69 and 79 may be twisted by screwing the male screw 66 into the female screw 16.

The double male connector 1 can be disconnected from the first female connector 6 and the second female connector 7 by performing the above-described operations in reverse order.

As described above, the double male connector 1 of Embodiment 1 includes the first male luer 10 and the female screw 16 that are suited to the ISO 80369-3 compliant first female connector 6 on one end and the second male luer 20 that is suited to the second female connector 7 on the other end. Accordingly, the feeding set having the female connector 6 that is compliant with ISO 80369-3 on its downstream end and the catheter having the conventional female connector 7 that is not compliant with ISO 80369-3 on its upstream end can be connected to each other via the double male connector 1.

The double male connector 1 and the first female connector 6 are connected to each other in compliance with ISO 80369-3, and therefore the connection therebetween achieves the liquid-tightness and connection strength equivalent to those of the connection between the male connector 901 (see FIGS. 11A and 11B) and the female connector 902 (see FIGS. 12A and 12B) of ISO 80369-3.

The double male connector 1 and the second female connector 7 are connected to each other with the liquid-tightness and connection strength equivalent to those of the connection between the second female connector 7 and a conventional male connector that is adapted to the second female connector 7.

### Embodiment 2

### Configuration of Double Male Connector

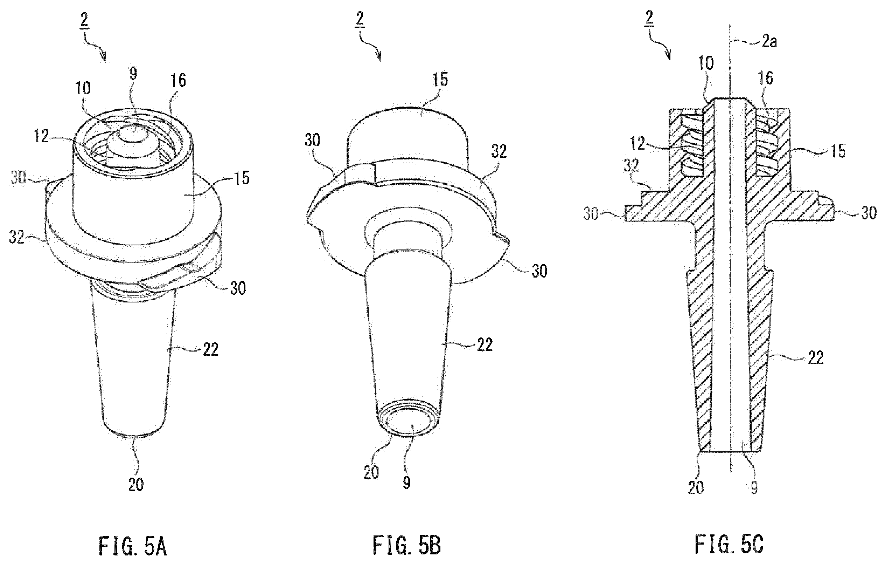

FIGS. 5A and 5B are perspective views of a double male connector 2 according to Embodiment 2 of the present invention. FIG. 5C is a cross-sectional view of the double male connector 2 taken along a plane containing a central axis 2a thereof. Elements that are the same as those of the double male connector 1 of Embodiment 1 are denoted by the same reference numerals, and their description is omitted. The following description of the double male connector 2 of Embodiment 2 focuses on the differences from Embodiment 1.

The double male connector 2 of Embodiment 2 includes a pair of engaging claws 30 protruding outward (i.e., in a radial direction away from the central axis 2a) between the first male luer 10 and the second male luer 20. A flange 32 that is continuous in the circumferential direction protrudes in an outward radial direction from an end of the tubular portion 15 on the second male luer 20 side. The pair of engaging claws 30 are provided on a cylindrical, outer circumferential surface of the flange 32. The engaging claws 30 extend in the circumferential direction (direction of rotation around the central axis 2a). As shown in FIG. 5B, those surfaces of the engaging claws 30 on the second male luer 20 side are flush with a surface of the flange 32 on the second male luer 20 side and a surface of the tubular portion 15 on the second male luer 20 side. As shown in FIG. 5A, a surface of each engaging claw 30 on the first male luer 10 side is formed by combining three inclined surfaces. However, the shape of each engaging claw 30 is not limited to that shown in FIGS. 5A to 5C and can be changed as appropriate. Preferably, the pair of engaging claws 30 are rotationally symmetrical with respect to the central axis 2a. The pair of engaging claws 30 are provided for engagement with a female connector into which the second male luer 20 is to be inserted. It is preferable that the pair of engaging claws 30 have compatibility with an engaging claw (see FIG. 24 of Patent Document 1, for example) that is engageable with a known female connector. It is preferable that the entirety of the double male connector 2 including the engaging claws 30 is integrally molded as one component by using injection molding or other methods using the same material as that for the double male connector 1 of Embodiment 1.

The double male connector 2 of Embodiment 2 is otherwise the same as the double male connector 1 of Embodiment 1.

### Female Connector to be Connected to Double Male Connector

The first female connector 6 (FIGS. 2A and 2B) that has been described in Embodiment 1 may be connected to the first male luer 10 of the double male connector 2.

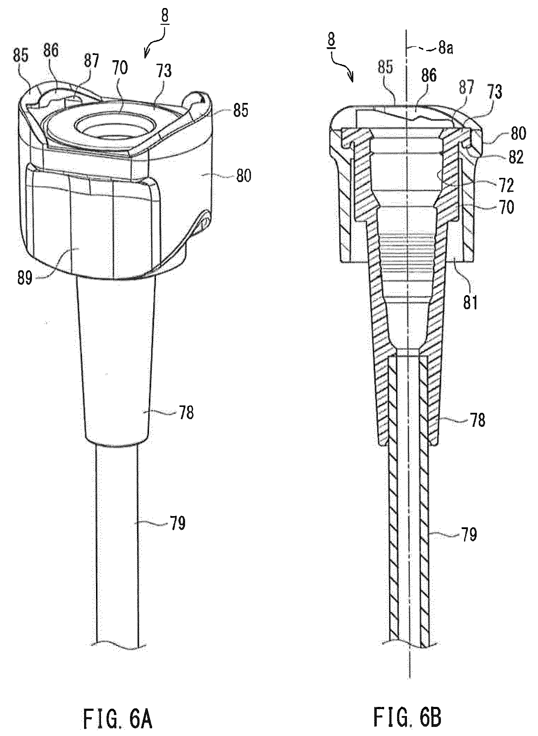

FIG. 6A is a perspective view of a second female connector 8 that is to be connected to the second male luer 20 of the double male connector 2, and FIG. 3B is a cross-sectional view of the second female connector 8 taken along a plane containing a central axis 8a thereof.

The second female connector 8 is obtained by externally fitting a handle 80 to the insertion portion 70 of the second female connector 7 that has been described in Embodiment 1.

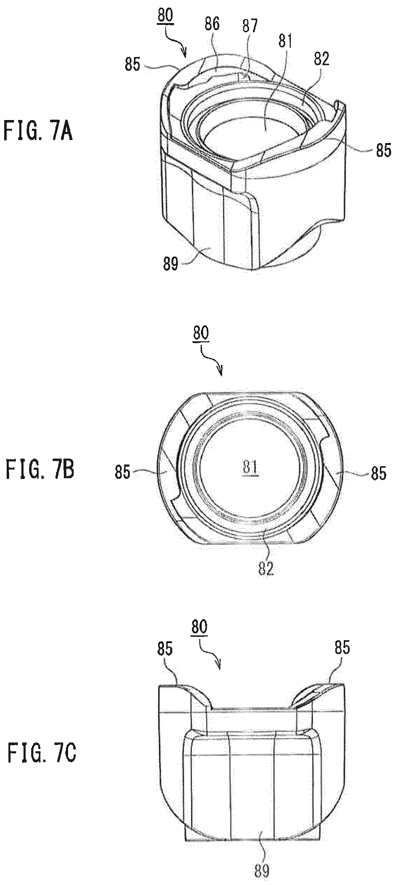

FIG. 7A is a perspective view of the handle 80, FIG. 7B is a top view of the handle 80, and FIG. 7C is a front view of the handle 80.

The handle 80 has a substantially tubular shape overall. An annular groove 82 is formed along an upper end edge of an opening 81 that passes through the handle 80 in a vertical direction. A pair of collar portions 85 protrude upward from respective positions on the outward side of the groove 82. Engaging walls 86 protrude toward each other from opposing inner circumferential surfaces of the respective collar portions 85. The engaging walls 86 extend in the circumferential direction. As shown in FIG. 6B, a lower surface of each engaging wall 86 is formed by combining three inclined surfaces so as to conform to the surface (see FIG. 5A) of each engaging claw 30 of the double male connector 2, on the first male luer 10 side. An end of each engaging wall 86 with respect to a longitudinal direction is closed by a stop portion 87, while the other end is open. The collar portions 85 and the engaging walls 86 are rotationally symmetrical with respect to a central axis of the handle 80. A pair of substantially flat grip portions 89 are provided on an outer circumferential surface of the handle 80.

Although there is no limitation on the material for the handle 80, it is preferable that the handle 80 is made of a hard material having a degree of stiffness that is high enough for that material to be regarded as being substantially undeformable. For example, a resin material such as polyacetal, polycarbonate, polystyrene, polyamide, polypropylene, polyethylene, unplasticized polyvinyl chloride, or ABS (acryl-butadiene-styrene copolymer) can be used. The handle 80 can be integrally formed as one component by using injection molding or other methods using such a resin material.

As shown in FIG. 6B, the insertion portion 70 is inserted into an opening 81 in the middle of the handle 80. The flange 73 of the insertion portion 70 is fitted into the groove 82 of the handle 80. The handle 80 is freely rotatable relative to the insertion portion 70.

The second female connector 8 and the tube 79 constitute a portion of a catheter, such as a PEG catheter or a transnasal catheter that have conventionally been used in enteral nutrition. The second female connector 8 serves as a female connector (see Patent Document 1) provided on an upstream end of the catheter. An end (downstream end) of the tube 79 opposite to the second female connector 8 is inserted into the patient.

### Manner in which Double Male Connector is Used

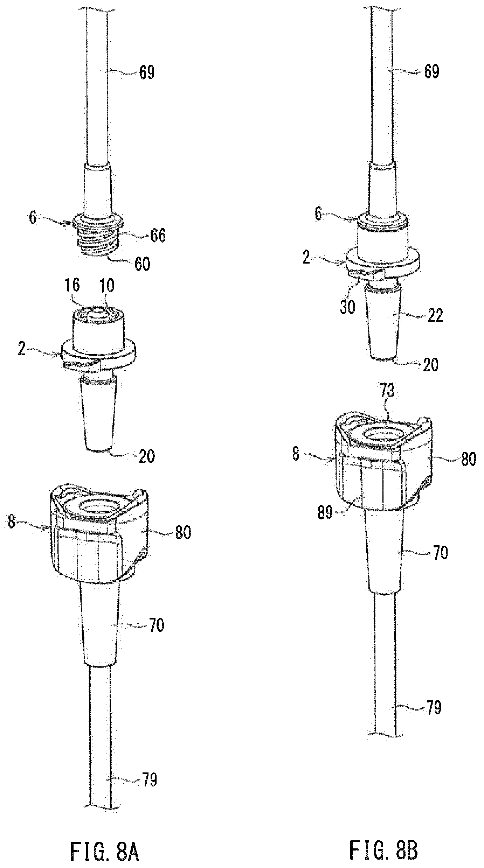

As shown in FIG. 8A, the double male connector 2 is used to connect the first female connector 6 provided on the downstream end of the feeding set and the second female connector 8 provided on the upstream end of the catheter to each other via the double male connector 2. Hereinafter, a manner in which the double male connector 2 is used will be described.

First, the double male connector 2 and the first female connector 6 are connected to each other. The method for connecting the double male connector 2 and the first female connector 6 to each other is the same as that of Embodiment 1. As shown in FIG. 8B, the double male connector 2 and the first female connector 6 are connected to each other in a liquid-tight manner.

Then, the second male luer 20 of the double male connector 2 is inserted into the insertion portion 70 of the second female connector 8. Since the insertion portion 70 has rubber elasticity, the insertion portion 70 is widened and stretched in the circumferential direction by the second tapered surface 22 of the second male luer 20 as the second male luer 20 is inserted into the insertion portion 70. It is possible to easily hold the second female connector 8 by gripping the grip portions 89 of the handle 80.

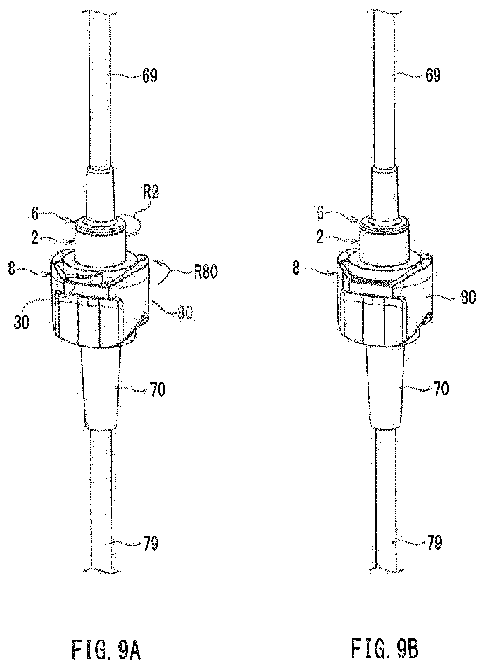

As shown in FIG. 9A, the second male luer 20 is inserted deep into the insertion portion 70 to such an extent that the engaging claws 30 of the double male connector 2 approach or come into contact with the flange 73 of the insertion portion 70. The direction in which the pair of engaging claws 30 oppose each other and the direction in which the pair of engaging walls 86 (see FIGS. 6A and 7A) oppose each other are substantially perpendicular to each other. In this state, the double male connector 2 and the handle 80 are rotated in opposite directions (arrows R2 and R80) relative to each other. The engaging claws 30 of the double male connector 2 engage with the corresponding engaging walls 86 formed in the collar portions 85 of the handle 80. The double male connector 2 and the handle 80 are rotated until an end of each engaging claw 30 abuts against the corresponding stop portion 87 (see FIG. 7A). Thus, as shown in FIG. 9B, the double male connector 2 and the second female connector 8 are connected to each other in a liquid-tight manner.

The female screw 16 surrounding the first male luer 10 and the male screw 66 of the first female connector 6 are right-handed screws that are compliant with ISO 80369-3. As shown in FIG. 7A, the stop portions 87 of the handle 80 are provided such that the engaging claws 30 abut against the corresponding stop portions 87 when the double male connector 2 is rotated in a clockwise direction (i. e., rotated to the right) when viewed from the double male connector 2 side. Therefore, in the state shown in FIG. 9A, even if the worker holds and rotates the first female connector 6, instead of the double male connector 2, and the handle 80 in opposite directions relative to each other, the screwing engagement between the female screw 16 of the double male connector 2 and the male screw 66 of the first female connector 6 does not become loose.

Conversely, it is also possible to first connect the double male connector 2 and the second female connector 8 to each other and then connect the double male connector 2 and the first female connector 6 to each other. However, with this connection method, in a state in which the first female connector 6 and the second female connector 8 are connected to each other via the double male connector 2 as shown in FIG. 9B, the tubes 69 and 79 may be twisted by screwing the male screw 66 into the female screw 16.

Alternatively, it is also possible to simultaneously screw the male screw 66 into the female screw 16 and engage the engaging claws 30 with the engaging walls 86, by inserting the first male luer 10 into the insertion portion 60 of the first female connector 6, inserting the second male luer 20 into the insertion portion 70 of the second female connector 8, and in this state, holding and rotating the first female connector 6 and the handle 80 in opposite directions relative to each other.

The double male connector 2 can be disconnected from the first female connector 6 and the second female connector 8 by performing the above-described operations in reverse order.

As described above, the double male connector 2 of Embodiment 2 includes the first male luer 10 and the female screw 16 that are suited to the ISO 80369-3 compliant first female connector 6 on one end, and includes the second male luer 20 and the pair of engaging claws 30 that are suited to the second female connector 8 on the other end. Accordingly, a feeding set having the female connector 6 that is compliant with ISO 80369-3 on its downstream end and a catheter having the conventional female connector 8 that is not compliant with ISO 80369-3 on its upstream end can be connected to each other via the double male connector 2.

Since the double male connector 2 and the first female connector 6 are connected to each other in compliance with ISO 80369-3, the connection therebetween achieves the liquid-tightness and connection strength equivalent to those of the connection between the male connector 901 (see FIGS. 11A and 11B) and the female connector 902 (see FIGS. 12A and 12B) of ISO 80369-3.

The double male connector 2 and the second female connector 8 are connected to each other with the liquid-tightness and connection strength equivalent to those of the connection between the second female connector 8 and a conventional male connector that is adapted to the second female connector 8. The double male connector 2 of Embodiment 2 can achieve higher connection strength than Embodiment 1 because of the engagement of the engaging claws 30 with the second female connector 8.

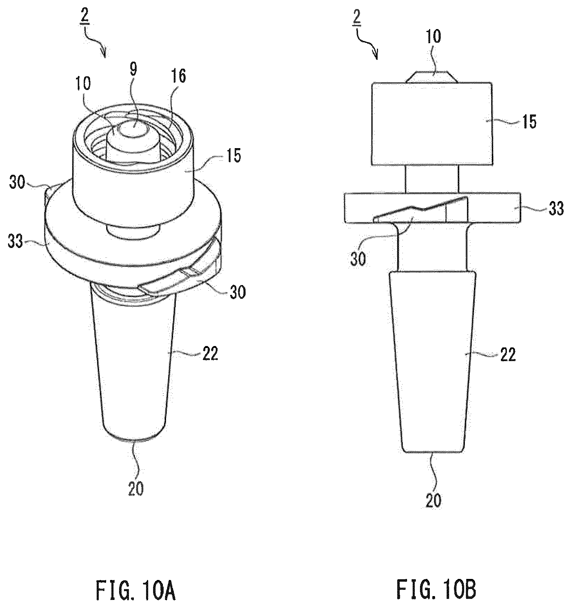

The engaging claws 30 of Embodiment 2 described above are provided on the flange 32 protruding from the tubular portion 15; however, depending on the outer diameter of the tubular portion 15, the engaging claws 30 may be directly formed on the outer circumferential surface of the tubular portion 15 without forming the flange 32. Alternatively, as shown in FIGS. 10A and 10B, it is also possible to form a disc-shaped flange 33 that is separate from the tubular portion 15 between the tubular portion 15 and the second male luer 20, and form the pair of engaging claws 30 on an outer circumferential surface of this flange 33.

The second male luer 20 of the double male connector 2 can be connected to the second female connector 7 that has been described in Embodiment 1. In this case, the engaging claws 30 are not used, but the double male connector 2 can be connected to the second female connector 7 with the liquid-tightness and connection strength equivalent to those described in Embodiment 1.

Embodiments 1 and 2 above have been described by way of example only. The present invention is not limited to Embodiments 1 and 2 above and can be changed as appropriate.

In Embodiments 1 and 2 above, the insertion portion 70 of the second female connector into which the second male luer 20 is inserted has rubber elasticity and is stretched in the circumferential direction as a result of the insertion of the second male luer 20. However, the second male luer 20 of the present invention can also be connected to a second female connector including an insertion portion 70 that is made of a substantially undeformable hard material. For example, if the second tapered surface 22 of the second male luer 20 is formed to conform to the tapered surface 72 (without the ribs 75) of the inner circumferential surface of that insertion portion 70 of the second female connector, the second male luer 20 can be connected to the insertion portion 70 of the second female connector in a liquid-tight manner. Alternatively, if the second male luer 20 is formed of a soft material having rubber elasticity, the second male luer 20 deforms appropriately in accordance with the tapered surface 72 and the ribs 75 on the inner circumferential surface of the insertion portion 70 of the second female connector, and thus the second male luer 20 can be connected to the insertion portion 70 of the second female connector in a liquid-tight manner. In the case of the double male connector 2 in which the second male luer 20 is composed of a soft material and the other portions are composed of a hard material, the entirety of the double male connector 2 can be integrally formed as one component by using coinjection molding, although there is no limitation to this method.

In Embodiments 1 and 2 above, the first male luer 10 and the second male luer 20 are integrally formed; however, for example, it is also possible to form the first male luer 10 and the second male luer 20 as separate components, and connect these components to each other via a pliable tube.

### Industrial Applicability

Although there is no limitation on the field of application of the present invention, the present invention is applicable to a connector for connecting an ISO 80369-3 compliant female connector and a conventionally used female connector to each other. In the foregoing embodiments, the case where the present invention is applied to the field of enteral nutrition has been described. However, the present invention is also applicable to fields other than the field of medicine (the fields of food, chemistry, and the like), not to mention fields in medicine other than enteral nutrition.

### List of Reference Numerals

- 1, 2: Double male connector
- 9: Flow channel
- 10: First male luer
- 12: First tapered surface
- 15: Tubular portion
- 16: Female screw
- 20: Second male luer
- 22: Second tapered surface
- 30: Engaging claw

## Claims

1. A double male connector comprising a first male luer on one end and a second male luer on another end, the first male luer and the second male luer being in communication with each other,
wherein an outer circumferential surface of the first male luer constitutes a first tapered surface whose outer diameter decreases toward a leading end of the first male luer,
an outer circumferential surface of the second male luer constitutes a second tapered surface whose outer diameter decreases toward a leading end of the second male luer,
the double male connector further comprises a tubular portion surrounding the first male luer, and
a female screw is formed on an inner circumferential surface of the tubular portion.

2. The double male connector according to claim 1, wherein the first tapered surface and the female screw are compliant with ISO 80369-3.

3. The double male connector according to claim 1 or 2, wherein the entirety of the double male connector is integrally formed as one component.

4. The double male connector according to any one of claims 1 to 3, further comprising a pair of engaging claws protruding outward between the first male luer and the second male luer.

5. The double male connector according to claim 4, wherein when the second male luer is inserted into a female connector, the pair of engaging claws engage with the female connector.
